# FASCICULE DE BREVET EUROPEEN

(11) **EP 1 064 931 B1**
(45) Date de publication et mention de la délivrance du brevet: **15.12.2004**
(21) Numéro de dépôt: 00401620.0
(22) Date de dépôt: 08.06.2000
(51) Int. Cl.: A61K 7/48

(54) **Composition cosmétique comprenant au moins un hydroxystilbene et de l'acide ascorbique**
Kosmetisches Mittel enthaltend mindestens einen Hydroxystilben und Ascorbinsäure
Cosmetic composition containing at least one hydroxystilbene and ascorbic acid

(30) Priorité: 02.07.1999 FR 9908570
(43) Date de publication de la demande: 03.01.2001
(73) Titulaire: L'OREAL, 75008 Paris (FR)
(72) Inventeur: Breton, Lionel, 78000 Versailles (FR); Liviero, Christel, 75008 Paris (FR)
(74) Mandataire: Galup, Cédric Olivier Nicolas

(56) Documents cités:
- EP-A- 0 773 020
- GB-A- 2 317 561
- STN, Serveur de Bases de Données, Karlsruhe, DE, Fichier CAPLUS, AN=1996:548009 * résumé * XP002136068 & JP 08 175960 A (KAO CORP) 9 juillet 1996 (1996-07-09)
- STN, Serveur de Bases de Données, Karlsruhe, DE, Fichier CAPLUS, AN=1997:387289 XP002136069

## Description

L'invention se rapporte à une composition comprenant au moins un hydroxystilbène en association avec de l'acide ascorbique. La composition est particulièrement une composition cosmétique raffermissante.

En particulier, la composition de l'invention est destinée à stimuler la restructuration de la peau et/ou des muqueuses par la stimulation de la prolifération des fibroblastes du derme.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même de différentes protéines extracellulaires parmi lesquelles figure notamment les fibres de collagène, l'élastine et différentes glycoprotéines. L'ensemble de ces composants extracellulaires est synthétisé par le fibroblaste. On trouve également dans le derme des leucocytes, des mastocytes ou encore des macrophages tissulaires. Enfin le derme contient des vaisseaux sanguins et des fibres nerveuses.

Le fibroblaste, de par son activité dans la synthèse des protéines extracellulaires matricielles (protéoglycannes, fibres de collagène et autres glycoprotéines de structure) est l'acteur principal de l'élaboration structurelle du derme.

Les fibres de collagène assurent la solidité du derme. Elles sont très résistantes mais sensibles à certaines enzymes appelées généralement collagénases. Dans le derme, les fibres de collagène sont constituées de fibrilles scellées les unes aux autres, formant ainsi plus de dix types de structures différentes. La structure du derme est en grande partie due à l'enchevêtrement des fibres de collagène tassées. Les fibres de collagène participent à la tonicité de la peau. Les fibres de collagènes sont régulièrement renouvelées mais ce renouvellement diminue avec l'âge ce qui entraîne notamment un amincissement du derme. Cet amincissement du derme est également dû à des causes pathologiques comme par exemple l'hypersécrétion d'hormones corticoïdes, certaines maladies (syndrome de Marfan, syndrome de Ehlers-Danlos) ou encore des carences vitaminiques (scorbut). Il est également admis que des facteurs extrinsèques comme les rayons ultraviolets, le tabac ou certains traitements (Acide rétinoïque et dérivés, glucocorticoïdes, vitamine D et dérivés par exemple) ont également un effet sur la peau et sur son taux de collagène.
Une dégradation des fibres de collagène entraîne notamment l'apparition d'une peau flasque et ridée que l'être humain, préférant l'apparence d'une peau lisse et tendue, cherche depuis toujours à combattre.

Par ailleurs, à la ménopause, les principales modifications concernant le derme sont une diminution du taux de collagène et de l'épaisseur dermique. Cela entraîne chez la femme ménopausée un amincissement de la peau. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée. Il est démontré que les femmes perdent progressivement leur taux de collagène par an après la ménopause et que 30% du taux global est perdu dans les cinq premières années postménopausiques.

Il est donc important de pouvoir disposer de produits dont les effets visent à maintenir le taux de collagène dans la peau et lui maintenir une apparence lisse et tendue.

Une des voies pour aboutir à ce résultat consiste donc à stimuler la prolifération des fibroblastes et en conséquence du fait de l'augmentation de la quantité de cellules sécrétrices dans le derme à maintenir voire renforcer la synthèse du collagène.

A cet égard la demanderesse a de manière surprenante et inattendue découvert que l'association d'au moins un hydroxystilbène et de l'acide ascorbique ou de l'un de ses analogues, présente la propriété de stimuler la prolifération des fibroblastes du derme dans des proportions plus importantes que celles raisonnablement attendues de la simple addition des effets de chacun de ces composants pris séparément.

Les hydroxystilbènes sont des composés répondant à la formule générale I : 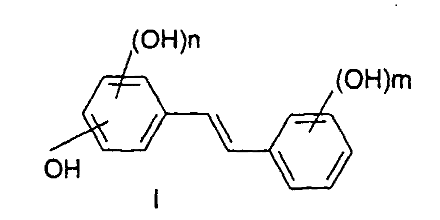 dans laquelle n est un nombre entier compris entre 0 et 4 inclusivement et m est un nombre entier compris entre 0 et 5 inclusivement.

Ces composés peuvent être sous une forme Cis ou Trans.

Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse et de carcinogenèse (JP 06024967), ou encore décrits comme antioxydants.

Parmi ces composés le resvératrol (ou 3, 5, 4'-trihydroxystilbène) est particulièrement étudié pour les activités ci-dessus décrites principalement parce qu'il s'agit d'un composé naturel qui se retrouve dans la peau des grains de raisin et dans le vin. A cet égard on peut consulter la revue de Soleas et collaborateurs (Clinical Biochemistry, vol. 30, n°2, pp. 91-113, 1997) qui résume parfaitement l'état des connaissances concernant ce composé et les hydroxystilbènes.

Par ailleurs, l'acide ascorbique (ou vitamine C) est connu pour stimuler la synthèse de collagène, en empêchant, en tant que co-facteur, l'auto-inactivation des enzymes lysine- et proline- hydroxylases et en augmentant la synthèse des ARNm de procollagènes. L'acide ascorbique (ou vitamine C) est également connu pour stimuler la synthèse de l'élastine de la peau. On peut citer à cet égard les brevets US 5801192, US 4983382 et EP 0717983. On peut également citer un article intitulé "Pola to incorporate vitamin C in new cosmetics line for skin care" du Japan Economic Journal du 5 juin 1984 (page 15). Ainsi, il a été décrit que l'acide ascorbique utilisé dans des compositions cosmétiques permet de traiter notamment les rides (Fragrance Journal, Vol.8, n°6(45) (1980) pp38-43, "Cosmetic and vitamin -action and safety to dermatology").

Ainsi, l'invention a donc pour objet une composition comprenant, dans un milieu physiologiquement acceptable, au moins un hydroxystilbène associé à l'acide ascorbique ou à l'un de ses analogues, caractérisée par le fait que l'hydroxystilbène est présent en une quantité pondérale allant de 0,005% à 5% du poids total de la composition et par le fait que l'acide ascorbique ou l'un de ses analogues est présent en une quantité pondérale allant de 3% à 10% du poids total de la composition, ainsi que ses utilisations.
Particulièrement, la composition est destinée à stimuler la prolifération des fibroblastes du derme.

Parmi les hydroxystilbènes, on peut citer les mono, di, tri, tétra, penta, hexa, hepta, octo, nonahydroxystilbènes, ou encore leurs dérivés hydroxyalkylés.

Selon l'invention les hydroxystilbènes peuvent être utilisés seuls ou en mélanges de toute nature et peuvent être d'origine naturelle ou synthétique.

Le hydroxystilbène utilisable selon l'invention est choisi parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène ou resvératrol.

Les analogues de l'acide ascorbique sont, plus particulièrement, ses sels, tels que notamment l'ascorbate de sodium, l'ascorbylphosphate de magnésium ou de sodium, ses esters, tels que notamment ses esters acétique, propionique, palmitique, cinnamique ou ferrulique ou ses sucres, tels que notamment l'acide ascorbique glycosilé.

L'acide ascorbique est généralement sous forme L, car il est habituellement extrait de produits naturels.

Selon l'invention l'acide ascorbique et/ou ses analogues peuvent être utilisés seuls ou en mélanges de toute nature et peuvent être d'origine naturelle ou synthétique.

Particulièrement, la composition de l'invention est utilisée en application topique sur la peau et/ou les muqueuses.

On a vu précédemment que le collagène est impliqué dans la solidité du derme, donc dans la fermeté de la peau et/ou des muqueuses et que les fibroblastes sont responsables de la synthèse des protéines de la matrice extracellulaire du derme, notamment du collagène.

Ainsi, un des aspects de l'invention est donc de proposer une composition comprenant au moins un hydroxystilbène en association avec de l'acide ascorbique, la composition étant destinée à traiter, de manière préventive ou curative, les signes cutanés du vieillissement, plus particulièrement à traiter, de manière préventive ou curative, la peau flasque et/ou ridée.

De préférence, la composition est destinée à diminuer les signes cutanés du vieillissement, plus particulièrement à diminuer l'apparence de la peau flasque et/ou ridée.

Un autre aspect de l'invention est de proposer une composition comprenant au moins un hydroxystilbène en association avec de l'acide ascorbique ou l'un de ses analogues destinée à stimuler le raffermissement de la peau.

Selon aussi un autre aspect, l'invention a pour objet une composition comprenant au moins un hydroxystilbène en association avec de l'acide ascorbique ou l'un de ses analogues destinée à favoriser le lissage de la peau et/ou à tendre la peau.

Selon encore un autre aspect, l'invention a pour objet une composition comprenant au moins un hydroxystilbène en association avec de l'acide ascorbique ou l'un de ses analogues destinée à combattre les effets cutanés de la ménopause, plus particulièrement les effets de la ménopause sur le collagène et/ou les fibroblastes.

Les quantités d'hydroxystilbène et d'acide ascorbique ou de ses analogues utilisables selon l'invention dépendent bien évidemment de l'effet recherché.

A titre d'exemple la quantité pondérale d'hydroxystilbène utilisable selon l'invention peut aller par exemple de 0,001 % à 10 % et de préférence de 0,005 % à 5 % du poids total de la composition.

Pour donner un ordre de grandeur, la quantité pondérale d'acide ascorbique ou de ses analogues utilisables selon l'invention représente de 0,001% à 20% du poids total de la composition, préférentiellement de 0,1% à 15% du poids total de la composition et avantageusement de 3% à 10% du poids total de la composition.

Dans la composition de l'invention, le rapport pondéral entre l'hydroxystilbène et l'acide ascorbique est compris entre 5.10⁻⁵ et 10⁺⁴ et de préférence compris entre 10⁻³ et 2.

En outre, la composition de l'invention est utilisée pendant un temps suffisant pour obtenir les effets attendus selon l'invention. Pour donner un ordre de grandeur, cette durée peut être au minimum de 3 semaines, mais peut être aussi de plus de 4 semaines, voire de plus de 8 semaines.

La composition est de préférence destinée à un usage cosmétique ou dermatologique, avantageusement cosmétique.

La composition de l'invention destinée à une application topique contient un milieu physiologiquement acceptable, c'est-à-dire compatible avec la peau y compris le cuir chevelu, les muqueuses et/ou les yeux et peut constituer notamment une composition cosmétique ou dermatologique.

L'invention a en outre pour objet l'utilisation dans une composition ou pour la préparation d'une composition de l'association d'au moins un hydroxystilbène et de l'acide ascorbique ou de l'un de ses analogues, pour traiter, de manière préventive ou curative, les signes cutanés du vieillissement, plus particulièrement pour traiter, de manière préventive ou curative, la peau flasque et/ou ridée.

De préférence, la composition est destinée à diminuer les signes cutanés du vieillissement, plus particulièrement à diminuer l'apparence de la peau flasque et/ou ridée.

L'invention a encore pour objet l'utilisation dans une composition ou pour la préparation d'une composition de l'association d'au moins un hydroxystilbène et de l'acide ascorbique ou de l'un de ses analogues dans une composition raffermissante.

L'invention a également pour objet l'utilisation dans une composition ou pour la préparation d'une composition de l'association d'au moins un hydroxystilbène et de l'acide ascorbique ou de l'un de ses analogues, pour le lissage de la peau ou pour tendre la peau.

L'invention a en outre pour objet l'utilisation dans une composition ou pour la préparation d'une composition de l'association d'au moins un hydroxystilbène et de l'acide ascorbique ou de l'un de ses analogues pour stimuler la prolifération des fibroblastes du derme.

L'invention a enfin pour objet l'utilisation dans une composition ou pour la préparation d'une composition de l'association d'au moins un hydroxystilbène et de l'acide ascorbique ou de l'un de ses analogues pour combattre les effets cutanés de la ménopause, plus particulièrement les effets de la ménopause sur les fibroblastes.

La composition selon l'invention comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant.

De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser en association avec l'hydroxystilbène utilisé selon l'invention des composés choisis parmi
- les hormones végétales ;
- les agents antagonistes de calcium, comme le vérapamil et le Diltiazem ;
- des capteurs de radicaux OH, tels que le diméthylsulfoxyde ;
- des ouvreurs de canaux chlore ;
- des extraits de végétaux tels que ceux d'Iridacées, de Rosacées ou de soja, extraits pouvant alors contenir ou non des isoflavones ;
- des extraits de micro-organismes dont en particulier des extraits bactériens comme ceux de bactéries filamenteuses non photosynthétiques ;

A la liste ci-dessus, d'autres composés peuvent également être rajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoïdes, les acides eicosatétraénoïque et eicosatriénoïque ou leurs esters et amides, la vitamine D et ses dérivés.

Selon l'invention, on peut, entre autres, associer au moins un hydroxystilbène à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les agents modulant l'adhésion bactérienne sur la peau et /ou les muqueuses tels que le miel, notamment le mile d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase ou encore les inhibiteurs de canaux sodiques, composés décrits comme étant actifs dans le traitement des peaux sensibles et comme présentant des effets anti-irritants, en particulier vis-à-vis de composés irritants éventuellement présents dans les compositions.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu) et leurs mélanges.

On peut aussi ajouter des actifs antirides, et notamment des produits tenseurs tels que les protéines végétales et leurs hydrolysats, en particulier l'extrait de protéines de soja vendue sous le nom d'Eleseryl® par la société LSN ou le dérivé d'avoine vendu sous la dénomination Reductine® par la société Silab.

La peau étant constituée de bien d'autres composants que le collagène et les fibroblastes, il s'avère intéressant, lorsque l'on utilise l'association d'un hydroxystilbène et de l'acide ascorbique selon l'invention, de favoriser en même temps la synthèse de ces autres composants comme par exemple les lipides et/ou de favoriser la prolifération d'autres composantes cellulaires comme par exemple les kératinocytes.

Ainsi, l'invention a pour objet une composition cosmétique comprenant dans un milieu cosmétiquement acceptable l'association d'au moins un hydroxystilbène et de l'acide ascorbique ou de l'un de ses analogues et au moins un autre produit stimulant la synthèse des lipides et/ou la prolifération des kératinocytes.

A cet égard on peut citer comme produit stimulant la synthèse des lipides les hormones végétales, comme les auxines, ou des composés d'origine végétale, comme l'acide cinnamique et comme produit stimulant la prolifération des kératinocytes des composés d'origine végétale, comme le phloroglucinol.

Ainsi, les compositions selon l'invention peuvent comprendre en plus de l'hydroxystilbène et de l'acide ascorbique , de l'acide cinnamique ou ses dérivés et/ou une hormone végétale, particulièrement une auxine choisie parmi l'acide indolacétique (IAA), l'acide 4-chloroindole-3-acétique (4-CI-IAA), l'acide phénylacétique (PAA), l'acide indole-3-butyrique (IBA), l'acide 2,4-dichlorophenoxyacétique (2,4-D), l'acide α-naphtalèneacétique (α-NAA), l'acide β-naphtoxyacétique, l'indole éthanol, l'idole acétaldéhyde et l'indole acétonitrile et/ou un composé végétal comme le phloroglucinol.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

### Exemple 1 : Etude de l'effet du resvératrol sur la prolifération des fibroblastes du derme.

L'étude est faite par mesure de l'incorporation de thymidine radioactive dans des cultures de fibroblastes dermiques humains normaux.
Les cultures de fibroblastes sont effectuées selon les méthodes classiques de culture cellulaire, à savoir en milieu MEM/M199 vendu par la société Gibco, en présence de bicarbonate de sodium (1,87 mg/ml) de L-glutamine (2 mM), de pénicilline (50 Ul/ml) et de 10% de sérum de veau foetal (Gibco).

Le test est effectué sur des cultures de cellules à 80% de confluence en plaque de 24 trous. Le resvératrol, à la concentration de 1,25 µM et 5 µM, est mis en contact avec les cellules pendant 48 heures. Le marquage à la thymidine tritiée ([méthyl-3H]thymidine vendue par la société Amersham, 82 Ci/mmole) est effectué pendant 24 heures.
Le taux de thymidine tritiée incorporée est mesuré en fin de test par précipitation acide des protéines sur filtres et comptage en scintillation liquide.

Les résultats sont évalués par rapport à un témoin constitué par des cellules n'ayant pas été traitées.

Un témoin positif [EGF : Epidermal Growth Factor (facteur épidermal de croissance)] à 1,67.10⁻³ µM, connu pour stimuler la prolifération des fibroblastes est introduit dans le test comme référence.

Les résultats de ce test sont présentés dans le tableau suivant.

| Traitement | cpm | d.s | % | p |
|---|---|---|---|---|
| Non traité | 10320 | 1427 | 100 | - |
| EGF (1,67.10⁻³ µM) | 29214 (+18894) | 1825 | 283 (+183) | <0.001 |
| Vitamine C 20 µM | 13595 (+3275) | 1394 | 132 (+32) | <0.05 |
| Resvératrol 5,00 µM | 11365 (+1045) | 613 | 110 (+10) | >0.05 |
| Resvératrol 5 µM + Vit. C 20 µM | 18944 (+8624) | 331 | 184 (+84) | <0.001 |
| (...) : différence par rapport au témoin non traité cpm : coups par minutes. d.s. : déviation standard p : intervalle de confiance calculé selon la méthode de Dunett. | | | | |

Les résultats présentés dans le tableau ci-dessus montrent sans ambiguïté que l'association resvératrol/vitamine C stimule la prolifération des fibroblastes de manière nettement plus importante que ce que l'on pouvait prédire en additionnant les effets mesurés du resvératrol et de la vitamine C utilisés seuls. Un effet de synergie entre le resvératrol et la vitamine C sur la prolifération des fibroblastes existe lorsque les deux produits sont utilisés simultanément.

### Exemple 2 : Exemples de compositions selon l'invention. Ces compositions sont obtenues par les techniques habituelles couramment utilisées en cosmétique ou en pharmacie.

| Composition 1 : crème de soin | |
|---|---|
| Cire d'abeille | 1,50 % |
| Huile d'amandes d'abricot | 13,00 % |
| Parfum | 0,40 % |
| Resvératrol | 1,00 % |
| Acide ascorbique | 5,00 % |
| Xanthane | 0,50 % |
| Cyclopentadiméthylsiloxane | 5,00 % |
| Mono-di-palmitostéarate de sucrose | 3,00 % |
| Sesquistéarate de méthylglucose | 3,00 % |
| Acide stéarique | 1,00 % |
| Alcool cétylique | 3,00 % |
| Conservateurs | 0,30 % |
| Eau déminéralisée stérilisée qsp | 100,00 % |

| Composition 2 : huile corporelle | |
|---|---|
| Huile de vaseline | 47,99 % |
| Huile d'amandes d'abricot | 6,00 % |
| Parfum | 1,00 % |
| Resvératrol | 0,50 % |
| Acide ascorbique | 3,00 % |
| Cyclopenta diméthylsiloxane | 45,00 % |

| Composition 3 : lait démaquillant | |
|---|---|
| Palmitate d'éthyl-2 hexyle | 10,50 % |
| Fraction liquide de beurre de karité | 16,50 % |
| Conservateurs | 0,30 % |
| Parfum | 0,15 % |
| Resvératrol | 0,50 % |
| Acide ascorbique | 2,00 % |
| Hydroxyde de sodium | 0,04 % |
| Polymère carboxyvinylique | 0,20 % |
| Eau déminéralisée stérilisée | 69,80 % |
| Mélange de cétylstéarylglucoside et d'alcools cétylique et stéarylique | 2,50 % |

| Composition 4 : crème de soin | |
|---|---|
| Cire d'abeille | 1,50 % |
| Huile d'amandes d'abricot | 13,00 % |
| Conservateurs | 0,30 % |
| Parfum | 0,40 % |
| Triéthanolamine | 0,17 % |
| Resvératrol | 1,00 % |
| Acide ascorbique | 5,00 % |
| Acide beta-naphtoxy acetique | 0,01 % |
| Acide 2,4-dichlorophenoxyacetique | 0,01 % |
| Xanthane | 0,50 % |
| Cyclopenta diméthylsiloxane | 5,00 % |
| Mono-di-palmitostéarate de sucrose | 3,00 % |
| Sesquistéarate de méthylglucose | 3,00 % |
| Acide stéarique | 1,00 % |
| Alcool cétylique | 3,00 % |
| Eau déminéralisée stérilisée qsp | 100,00 % |

## Revendications

1. Utilisation dans une composition cosmétique de l'association d'au moins un hydroxystilbène et de l'acide ascorbique ou de l'un de ses analogues, ladite association étant destinée à stimuler la prolifération des fibroblastes du derme.

2. Utilisation selon la revendication 1, **caractérisée par le fait que** ladite association est destinée à stimuler le raffermissement de la peau.

3. Utilisation selon la revendication 1, **caractérisée par le fait que** ladite association est destinée à combattre les effets cutanés de la ménopause.

4. Utilisation dans une composition cosmétique de l'association d'au moins un hydroxystilbène et de l'acide ascorbique ou de l'un de ses analogues, ladite association étant destinée à combattre les effets de la ménopause sur les fibroblastes étant du derme.

5. Utilisation selon l'une quelconque des revendications 1 à 4, **caractérisée par le fait que** l'hydroxystilbène est choisi parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2,3',4,4',6-pentahydroxystilbène,
le 2,2',4,4',6,6'-hexahydroxystilbène.

6. Utilisation selon l'une quelconque des revendications 1 à 5, **caractérisée par le fait que** l'hydroxystilbène est du 3,4',5-trihydroxystilbène ou resvératrol.

7. Utilisation selon l'une quelconque des revendications 1 à 6, **caractérisée par le fait que** l'hydroxystilbène est présent en une quantité pondérale allant de 0,001% à 10% du poids total de la composition

8. Utilisation selon la revendication précédente, **caractérisée par le fait que** l'hydroxystilbène est présent en une quantité pondérale allant de 0,005% à 5% du poids total de la composition.

9. Utilisation dans une composition cosmétique de l'association d'au moins un hydroxystilbène et de l'acide ascorbique ou de l'un de ses analogues selon la revendication 1, ladite association étant destinée à traiter, de manière préventive ou curative, les signes cutanés du vieillissement, **caractérisée par le fait que** l'hydroxystilbène est présent en une quantité pondérale allant de 0,005% à 5% du poids total de la composition et **par le fait que** l'acide ascorbique ou l'un de ses analogues est présent en une quantité pondérale allant de 3% à 10% du poids total de la composition.

10. Utilisation selon la revendication précédente pour traiter, de manière préventive ou curative, la peau flasque et/ou ridée.

11. Utilisation dans une composition cosmétique de l'association d'au moins un hydroxystilbène et de l'acide ascorbique ou de l'un de ses analogues selon la revendication 1, ladite association étant destinée à favoriser le lissage de la peau et/ou à tendre la peau, **caractérisée par le fait que** l'hydroxystilbène est présent en une quantité pondérale allant de 0,005% à 5% du poids total de la composition et **par le fait que** l'acide ascorbique ou l'un de ses analogues est présent en une quantité pondérale allant de 3% à 10% du poids total de la composition.

12. Utilisation de l'association d'au moins un hydroxystilbène et de l'acide ascorbique ou de l'un de ses analogues pour la préparation d'une composition pharmaceutique destinée à stimuler la prolifération des fibroblastes du derme.

13. Utilisation selon l'une quelconque des revendications 1 à 12, **caractérisée par le fait que** la composition est utilisée en application topique sur la peau et/ou les muqueuses.

14. Composition comprenant dans un milieu physiologiquement acceptable au moins un hydroxystilbène associé à l'acide ascorbique ou à l'un de ses analogues, **caractérisée par le fait que** l'hydroxystilbène est présent en une quantité pondérale allant de 0,005% à 5% du poids total de la composition et **par le fait que** l'acide ascorbique ou l'un de ses analogues est présent en une quantité pondérale allant de 3% à 10% du poids total de la composition.

15. Composition selon la revendication précédente, **caractérisée par le fait qu'**elle est utilisée en application topique sur la peau et/ou les muqueuses.

16. Composition selon l'une quelconque des revendications 14 ou 15, **caractérisée par le fait que** l'hydroxystilbène est choisi parmi :
le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2,3',4,4',6-pentahydroxystilbène,
le 2,2',4,4',6,6'-hexahydroxystilbène.

17. Composition selon l'une quelconque des revendications 14 à 16, **caractérisée par le fait que** l'hydroxystilbène est du 3,4',5-trihydroxystilbène ou resvératrol.

## Patentansprüche

1. Verwendung der Kombination aus mindestens einem Hydroxystilben und Ascorbinsäure oder einer zu Ascorbinsäure analogen Verbindung in einer kosmetischen Zusammensetzung, wobei die Kombination dazu vorgesehen ist, die Proliferation der Fibroblasten der Dermis zu stimulieren.

2. Verwendung nach Anspruch 1, **dadurch gekennzeichnet, dass** die Kombination dazu vorgesehen ist, die Straffung der Haut zu fördern.

3. Verwendung nach Anspruch 1, **dadurch gekennzeichnet, dass** die Kombination dazu vorgesehen ist, die Wirkungen der Menopause auf die Haut zu bekämpfen.

4. Verwendung der Kombination aus mindestens einem Hydroxystilben und Ascorbinsäure oder einer zu Ascorbinsäure analogen Verbindung in einer kosmetischen Zusammensetzung, wobei die Kombination dazu vorgesehen ist, die Wirkungen der Menopause auf die Fibroblasten der Dermis zu bekämpfen.

5. Verwendung nach einem der Ansprüche 1 bis 4, **dadurch gekennzeichnet, dass** das Hydroxystilben ausgewählt ist unter:
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben, ,
2,2',4,4',6,6'-Hexahydroxystilben.

6. Verwendung nach einem der Ansprüche 1 bis 5, **dadurch gekennzeichnet, dass** es sich bei dem Hydroxystilben um das 3,4',5-Trihydroxystilben oder Resveratrol handelt.

7. Verwendung nach einem der Ansprüche 1 bis 6, **dadurch gekennzeichnet, dass** das Hydroxystilben in einer Menge von 0,001 bis 10 % des Gesamtgewichts der Zusammensetzung vorliegt.

8. Verwendung nach dem vorhergehenden Anspruch, **dadurch gekennzeichnet, dass** das Hydroxystilben in einer Menge von 0,005 bis 5 % des Gesamtgewichts der Zusammensetzung vorliegt.

9. Verwendung der Kombination aus mindestens einem Hydroxystilben und Ascorbinsäure oder einer zu Ascorbinsäure analogen Verbindung gemäß Anspruch 1 in einer kosmetischen Zusammensetzung, wobei die Kombination dazu vorgesehen ist, präventiv oder kurativ die Anzeichen der Hautalterung zu bekämpfen, **dadurch gekennzeichnet, dass** das Hydroxystilben in einer Gewichtsmenge von 0,005 bis 5 % des Gesamtgewichts der Zusammensetzung und die Ascorbinsäure oder die zu Ascorbinsäure analoge Verbindung in einer Gewichtsmenge von 3 bis 10 % des Gesamtgewichts der Zusammensetzung enthalten ist.

10. Verwendung nach dem vorhergehenden Anspruch zur präventiven oder kurativen Behandlung von schlaffer und/oder faltiger Haut.

11. Verwendung der Kombination aus mindestens einem Hydroxystilben und Ascorbinsäure oder einer zu Ascorbinsäure analogen Verbindung gemäß Anspruch 1 in einer kosmetischen Zusammensetzung, wobei die Kombination dazu vorgesehen ist, die Glättung der Haut zu fördern und/oder die Haut zarter zu machen, **dadurch gekennzeichnet, dass** das Hydroxystilben in einer Gewichtsmenge von 0,005 bis 5 % des Gesamtgewichts der Zusammensetzung und die Ascorbinsäure oder die zu Ascorbinsäure analoge Verbindung in einer Gewichtsmenge von 3 bis 10 % des Gesamtgewichts der Zusammensetzung enthalten ist.

12. Verwendung der Kombination aus mindestens einem Hydroxystilben und Ascorbinsäure oder einer zu Ascorbinsäure analogen Verbindung zur Herstellung einer pharmazeutischen Zusammensetzung, die dazu vorgesehen ist, die Proliferation der Fibroblasten der Dermis zu stimulieren.

13. Verwendung nach einem der Ansprüche 1 bis 12, **dadurch gekennzeichnet, dass** die Zusammensetzung topisch auf die Haut und/ oder die Schleimhäute angewendet wird.

14. Zusammensetzung, die in einem physiologisch akzeptablen Medium mindestens ein Hydroxystilben in Kombination mit Ascorbinsäure oder einer zu Ascorbinsäure analogen Verbindung enthält, **dadurch gekennzeichnet, dass** das Hydroxystilben in einer Gewichtsmenge von 0,005 bis 5 % des Gesamtgewichts der Zusammensetzung und die Ascorbinsäure oder die zu Ascorbinsäure analoge Verbindung in einer Gewichtsmenge von 3 bis 10 % des Gesamtgewichts der Zusammensetzung enthalten ist.

15. Zusammensetzung nach dem vorhergehenden Anspruch, **dadurch gekennzeichnet, dass** die Zusammensetzung topisch auf die Haut und/oder die Schleimhäute angewendet wird.

16. Zusammensetzung nach einem der Ansprüche 14 oder 15, **dadurch gekennzeichnet, dass** das Hydroxystilben ausgewählt ist unter:
4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4',5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben.

17. Zusammensetzung nach einem der Ansprüche 14 bis 16, **dadurch gekennzeichnet, dass** es sich bei dem Hydroxystilben um das 3,4',5-Trihydroxystilben oder Resveratrol handelt.

## Claims

1. Use in a cosmetic composition of a combination of at least one hydroxystilbene and of ascorbic acid or an analogue thereof, the said combination being intended to stimulate the proliferation of dermal fibroblasts.

2. Use according to Claim 1, **characterized in that** the said combination is intended to stimulate firming of the skin.

3. Use according to Claim 1, **characterized in that** the said combination is intended to combat the effects of the menopause on the skin.

4. Use in a cosmetic composition of a combination of at least one hydroxystilbene and of ascorbic acid or an analogue thereof, the said combination being intended to combat the effects of the menopause on dermal fibroblasts.

5. Use according to any one of Claims 1 to 4, **characterized in that** the hydroxystilbene is chosen from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene.

6. Use according to any one of Claims 1 to 5, **characterized in that** the hydroxystilbene is 3,4',5-trihydroxystilbene, or resveratrol.

7. Use according to any one of Claims 1 to 6, **characterized in that** the hydroxystilbene is present in an amount by weight ranging from 0.001% to 10% relative to the total weight of the composition.

8. Use according to the preceding claim, **characterized in that** the hydroxystilbene is present in an amount by weight ranging from 0.005% to 5% relative to the total weight of the composition.

9. Use in a cosmetic composition of a combination of at least one hydroxystilbene and of ascorbic acid or an analogue thereof according to Claim 1, the said combination being intended to preventively or curatively treat the signs of ageing of the skin, **characterized in that** the hydroxystilbene is present in an amount by weight ranging from 0.005% to 5% relative to the total weight of the composition and **in that** the ascorbic acid or an analogue thereof is present in an amount by weight ranging from 3% to 10% relative to the total weight of the composition.

10. Use according to the preceding claim, to preventively or curatively treat loose and/or wrinkled skin.

11. Use in a cosmetic composition of a combination of at least one hydroxystilbene and of ascorbic acid or an analogue thereof according to Claim 1, the said combination being intended to promote the smoothness of the skin and/or to make the skin taut, **characterized in that** the hydroxystilbene is present in an amount by weight ranging from 0.005% to 5% relative to the total weight of the composition and **in that** the ascorbic acid or an analogue thereof is present in an amount by weight ranging from 3% to 10% relative to the total weight of the composition.

12. Use of a combination of at least one hydroxystilbene and of ascorbic acid or an analogue thereof for the preparation of a pharmaceutical composition intended to stimulate the proliferation of dermal fibroblasts.

13. Use according to any one of Claims 1 to 12, **characterized in that** the composition is used in topical application on the skin and/or mucous membranes.

14. Composition comprising, in a physiologically acceptable medium, at least one hydroxystilbene combined with ascorbic acid or with an analogue thereof, **characterized in that** the hydroxystilbene is present in an amount by weight ranging from 0.005% to 5% relative to the total weight of the composition and **in that** the ascorbic acid or an analogue thereof is present in an amount by weight ranging from 3% to 10% relative to the total weight of the composition.

15. Composition according to the preceding claim, **characterized in that** it is used in topical application on the skin and/or mucous membranes.

16. Composition according to either of Claims 14 and 15, **characterized in that** the hydroxystilbene is chosen from:
4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene,
2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3',4,4',6-pentahydroxystilbene,
2,2',4,4',6,6'-hexahydroxystilbene.

17. Composition according to any one of Claims 14 to 16, **characterized in that** the hydroxystilbene is 3,4',5-trihydroxystilbene, or resveratrol.
